# EUROPEAN PATENT APPLICATION

(11) **EP 4 793 358 A2**
(43) Date of publication of application: **19.08.2026**
(21) Application number: 26180658.2
(22) Date of filing: 06.07.2021
(51) Int. Cl.: C12P 7/625

(54) **SOLUTION AND METHOD FOR THE OXIDATIVE LYSIS AND CONDITIONING OF POLYHYDROXYALKANOATE PRODUCING CELLS**

(30) Priority: 13.07.2020 US 202063051362 P
(62) Document number(s) of the earlier application(s) in accordance with Art. 76 EPC: 21842010.7 / 4 178 700
(71) Applicant: Newlight Technologies, Inc., Huntington Beach CA 92647 (US)
(72) Inventor: Coragliotti, Anna, Long Beach 90803 (US); Kimmel, Kenton, Long Beach 90804 (US); Sell, Garrett, Lakewood 90713 (US)
(74) Representative: Grünecker Patent- und Rechtsanwälte PartG mbB

(57) **Abstract**

The present specification generally relates to a method for the oxidative conditioning of a microbial cell wall, and its eventual lysis. In particular, the specification pertains to an oxidative lysis solution and methodology, wherein the selective lysis solution is capable of conditioning and lysing the cell wall of polyhydroxyalkanoate-producing microbes, while having a limited effect on the degradation of the polyhydroxyalkanoate (PHA) biopolymer solids contained therein, thereby allowing for the further high yield recovery, and purification of high molecular weight PHA biopolymer solids.

## Description

### CROSS REFERENCE TO OTHER APPLICATIONS

This application claims benefit of priority to U.S. Provisional Application No. 63/051,362, filed July 13, 2020, the disclosure of which is fully incorporated herein by reference

### BACKGROUND OF THE INVENTION

### 1. Field of the Invention:

The present specification generally relates to both a lysis solution and a method useful for conditioning and disrupting the cell wall of polyhydroxyalkanoate-producing microbes In particular, the specification pertains to a mild lysis solution comprising an oxidizing agent that is non-gaseous at room temperature having an oxidation-reduction potential (Eₒ) of more than than 1.4 volts, a pH between 3 and 11, and a molar mass of less than 250g/mol, wherein the lysis solution is capable of disrupting the cell wall of PHA-producing microbes having overall intercellular PHA concentrations of greater than 30% PHA when heated at a temperature above 60° C. The lysis solution and methodology disclosed herein has a limited effect on the degradation of the PHA biopolymer solids contained within the cell thus preserving the maximum original characteristics of the PHA biopolymer, and allowing for the further recovery, modification, and purification of the PHA biopolymer solids at high yields.

### 2. Description of the State of the Art:

In the first decade of this century, more plastic was produced than all the plastic in history up to the year 2000. The use of plastic materials on a large scale has represented a mark in the history of technological development; however, the increasing utilization of these materials is resulting in serious environmental problems. These materials take approximately 500 - 1,000 years to degrade naturally, meaning that virtually every piece of plastic ever made still exists in some shape or form. In the case of petrochemical-derived plastic resins, approximately 775 billion pounds worldwide are produced annually, and it is estimated that this number will continue to increase each year by approximately four percent. Of this annual worldwide production, it is estimated that approximately 10 percent of 77 billion pounds enters the earth's oceans on an annual basis, resulting in the deaths of thousands of seabirds and sea turtles, seals and other marine mammals each year after either ingesting the plastic or becoming entangled in it.

In view of these problems, the development of biodegradable plastic resins has received worldwide attention. Considering the relevance of these facts, the market potential for using these new materials is enormous. The applications for these biodegradable biopolymers in the market involve products, such as disposable materials, including but not limited to packaging, diapers; foodware, such as dishware, drinkware, and cutlery; cosmetic and agrochemical products; and medical and pharmaceutical articles, such as microencapsulating drugs for controlled release, medical sutures and fixation pins for bone fractures, due to their total biocompatibility and mild rejection from the receiving organism.

An important family of the biodegradable biopolymers is the polyhydroxyalkanoates (PHAs), which are polyesters naturally synthesized by over 300 different microorganisms, serving as natural energy reserves for the microbe. The commercial interest in the PHAs is directly related not only to the biodegradability and biocompatibility characteristics but also to their thermo-mechanical properties and production costs. These thermoplastic or elastic polyesters may be conveniently synthesized by cultivating a wide variety of microorganisms, bacteria in particular, in an aqueous medium on a carbon source, including sugars, alkanes, carbon dioxide, vegetable oils, organic acids, and alcohols. Depending on the microorganism, carbon source, nutrients and culture conditions the PHA is typically stored inside of the cell as discrete amorphous, water insoluble granules making it difficult to isolate and purify. The average molecular weight of PHAs typically range from about 100,000 - 500,000 g/mol and can account for up to 95% of dry cellular weight. However, molecular weights of considerably more than 1,000,000 g/mol are obtainable under special conditions.

Each discrete PHA granule, within the cell, is surrounded by a phospholipid monolayer membrane in which proteins, including the PHA synthase and degradase, are located. Other proteins (phasins) are presumed to be involved in stabilization of the amorphous hydrophobic PHA granules suspended in cell cytoplasm. It is this specific cell morphology that makes extracting and isolating PHA a challenging task. In fact, some of the most successful laboratory cell disruption techniques have no possibility of commercialization.

The majority of PHA recovery methods are performed using a solvent extraction process. Popular solvents are halogenated hydrocarbons such as chloroform and dichloromethane; however, since these halogen-containing hydrocarbons are hydrophobic solvents, a pre-extraction procedure, such as drying the cells in advance or otherwise, allowing the solvent to directly contact the intracellular PHA is required. As an alternative to solvent extraction, PHAs may be extracted from biomass using aqueous processing techniques, in which the polymer remains in a microparticulate state and the non-PHA cell mass (NPCM) is solubilized through mechanical, chemical, and/or enzymatic treatments.

Generally, the extraction methods typically utilized for the recovery and purification of PHA from a cell biomass can be classified as chemical, biological, mechanical, and physical methods independently or in combination. Regardless of whether a solvent or aqueous extraction methodology is used, the first step of isolating and purifying PHA is the lysing of the cell wall thus making the PHA accessible. Coty, V.F, in U.S. Pat. No. 3,275,610 discloses many methodologies such as, ultrasonic vibration, grinding, French pressing, freezing/thawing cycles and lysozyme treatment to accomplish this task. Spray or flash drying of the suspension of cells, as produced by culturing the microorganism in an aqueous medium on a suitable carbon and energy source, can also cause sufficient cell breakage to enable the PHA to be extracted from the cells as described in European Patent Application No. 15123. The PHA particles can then be easily removed from the solubilized material by centrifugation, filtration, flotation, washing or other known methods. Unfortunately, many of the cell disruption techniques used to gain access to the PHA biopolymer solids contained therein cause severe degradation of the molecular weight of the PHA biopolymer. The average molecular weight of PHAs typically range from about 100,000 - 500,000 g/mol; however, many of the lysis methods result in damage or degradation of the PHA biopolymer rending the material with reduced functionality.

Thus a need exists for both a mild lysis solution and methodology that selectively lyses or disrupts the cell walls of polyhydroxyalkanoate-producing microbes while preserving the maximum original characteristics of the PHA biopolymer, with high yield and efficiency.

### BRIEF SUMMARY OF THE INVENTION

Accordingly, one object of this invention is to provide a lysis solution useful for the selective disruption of a microbial cell wall when the microbe is heated at a temperature above 60° C.

Another object of the present invention is to provide an industrially feasible process for the selective disruption of a microbial cell wall allowing for the recovery, modification and purification of PHA biopolymer solids produced by PHA-containing cells.

Another object of the present invention is to heat deactivate enzymes present in a microbial biomass thereby preventing such enzymes from adversely interacting with the lysis solution comprising an oxidizing agent.

To achieve the foregoing and other objects and in accordance with the purposes of the present invention, as embodied and broadly described herein, one embodiment of this invention comprises adding an oxidizing agent to a heated suspension of PHA-containing microorganisms comprising greater than 30 percent of a PHA biopolymer to a final concentration of, by way of example, about 0.1 - 10%. The heated suspension of microorganisms comprising PHA biopolymer and non-polymer cell mass (NPCM) are maintained in contact with the oxidizing solution for a period of time and at a temperature sufficient to condition and lyse open said PHA-containing microorganisms thereby allowing for the later separation and recovery of the PHA biopolymer from the NPCM.

It is yet another object of the present invention to provide an enriched source of high molecular weight polyhydroxyalkanoate (PHA) for further purification wherein the enriched source of PHA is attained by heating a suspension of microorganisms containing PHA at a concentration of above 30% of the cells's dry weight and then exposing said heated suspension of PHA-containing microorganisms to an oxidizing agent for a period of time and at a temperature sufficient to condition and lyse said microorganisms thereby releasing PHA from the microorganism.

It is yet another object of the present invention wherein the PHA biopolymer producing microbial cells used are bacterial cells. In one embodiment said PHA biopolymer producing microbial cells are selected from a strain selected from the group *Ciipriavidus necatori,* methanotrophs from the genus *Methylocystis, Methylsinus, Methylococcus, Halomonas, Zobellella, Pseudomonas, Bacillus, Chromobacterium,* or any combinations thereof. Other PHA-accumulating strains such as yeast, fungi or other biopolymer producing microbial cells or mixed cultures could also be used.

It is yet another object of the present invention to use a carbon containing gas such as methane, carbon dioxide or a combination thereof as the carbon source during the fermentation for the PHA biopolymer producing microbial cell.

It is yet another object of the present invention to produce a PHA biopolymer comprising poly(3-hydroxypropionate) (PHP or P3HP), poly(3-hydroxybutyrate) (PHB or P3HB), poly(4-hydroxybutyrate) (P4HB), poly(3-hydroxyvalerate) (PHV or P3HV), poly(4-hydroxyvalerate) (P4HV), poly(5-hydroxyvalerate) (P5HV), poly(3-hydroxyhexanoate) (PHHx or P3HHx), poly(3-hydroxyoctanoate) (PHO, or P3HO), poly(3-hydroxydecanoate) (PHD or P3HD), poly(3-hydroxyundecanoate) (PHU, P3HU), or other short- or medium-chain length, saturated or unsaturated PHAs; or polylactic acid (PLA); or their copolymers or any combinations thereof.

Additional embodiments and features are set forth in the description that follows, and in part will become apparent to those skilled in the art upon examination of the specification or may be learned by the practice of the disclosed embodiments. The features and advantages of the disclosed embodiments may be realized and attained by means of the instrumentalities, combinations, and methods described in the specification.

### DETAILED DESCRIPTION OF THE INVENTION

The present invention is related to a selective lysis solution and process, proven to be industrially feasible, useful for the selective lysis or disruption of the cell walls of polyhydroxyalkanoates (PHA) producing microbes while preserving at maximum the original characteristics of the PHA biopolymer, with high yield and efficiency.

As used herein the term "PHA biopolymers" refers to poly(3-hydroxypropionate) (PHP or P3HP), poly(3-hydroxybutyrate) (PHB or P3HB), poly(4-hydroxybutyrate) (P4HB), poly(3-hydroxyvalerate) (PHV or P3HV), poly(4-hydroxyvalerate) (P4HV), poly(5-hydroxyvalerate) (P5HV), poly(3-hydroxyhexanoate) (PHHx or P3HHx), poly(3-hydroxyoctanoate) (PHO, or P3HO), poly(3-hydroxydecanoate) (PHD or P3HD), poly(3-hydroxyundecanoate) (PHU, P3HU), or other short- or medium-chain length, saturated or unsaturated PHAs; or polylactic acid (PLA); or their copolymers or any combinations thereof.

As used herein the term "fermentation" or "fermentation process" refers to any fermentation process or any process comprising a fermentation step. A fermentation process includes, without limitation, fermentation processes used to produce PHAs and are well known in the art. Examples of such can be found in U.S. Patents 7,579,176 and 9,850,508 issued to Herrema, et al.*,* all of which are incorporated herein by reference.

As used herein the term "fermentation media" or "fermentation medium" refers to the environment in which the fermentation is carried out and which includes the fermentation substrate, that is, the carbon source that is metabolized by the fermenting microorganism. The fermentation media, including fermentation substrate and other raw materials used in the fermentation process may be processed prior to or simultaneously with the fermentation process. Accordingly, the fermentation media can refer to the media before the fermenting microorganisms are added, as well as the media which comprises the fermenting microorganisms.

As used herein the term "fermenting microorganism" refers to any microorganism suitable for use in a desired fermentation process. Suitable fermenting microorganisms according to the invention are able to ferment, *i.e.,* convert, methane, carbon dioxide, sugars, alkanes, vegetable oils, organic acids, and alcohols, directly or indirectly into the PHA. Sources from which PHA is extracted *via* the process of the present invention include single-cell organisms such as bacteria or fungi and higher organisms such as plants (herein collectively referred to as "biomass"). While such biomass could be genetically manipulated species, they are preferably wild-type organisms specifically selected for the production of a specific PHA of interest. Bacteria useful in the present invention include any bacteria which naturally produce PHA. To date, *Cupriavidus necator* (formerly known as *Wautersia eutropha, Ralstonia eutropha* and Alcaligenes eutrophus) is the most extensively studied microorganism for the cost-effective production of PHA. Numerous other strains such as *Bacillus megaterium, Bacillus cereus* SPV, *Sinorhizobium meliloti, Azotobacter spp, Pseudomonas putida* KT2440 and *Metylobacterium spp,* and *Methylococcus spp* are also gaining attention for PHA production. These bacteria can accumulate up to 30-90% of their weight as PHB under limiting nitrogen substrate and in the presence of an abundant source of carbon such as but not limited to methane, carbon dioxide, sugars, alkanes, vegetable oils, organic acids, and alcohols. For further examples of such bacteria the following articles and patents are incorporated herein by reference - NOVEL BIODEGRADABLE MICROBIAL POLYMERS, E. A. Dawes, ed., NATO ASI Series, Series E: Applied Sciences--Vol. 186, Kluwer Academic Publishers (1990); Herrema, et. al., (U.S. Pat. No. 7,579,176); Shiotani, et. al., (U.S. Pat. No. 5,292,860,) ; and, Peoples, et. al., (U.S. Pat. No. 5,250,430).

As used herein the term "oxidizing agents" refers to compounds that are non-gaseous at room temperature having an oxidation-reduction potential (Eo) of more than 1.4 volts, a pH of more than 3 and less than 11, and a molar mass of less than 250g/mol. Examples of such oxidizing agents includes but is not limited to hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.

The general conditions of growth and fermentation are well known in the art. For carrying out the process according to the invention, at least part of the fermentation solution or the water is first removed from the fermented, aqueous cell suspension. Examples of separation processes which can be employed here are decanting, centrifugation, spray drying, evaporation, and filtration of the biomass from the fermentation medium. It is preferred to optionally remove part of the fermentation medium from the cell mass by centrifugation, preferably with the aid of a separator so that the resulting biomass slurry contains a final biomass concentration of approximately 5-10 percent, 10 - 16 percent, 16-20 percent, 20-25 percent, or 25-35 percent solids, or preferable targeted percentages therein.

One of the advantages of the process according to the invention is that there is no need to pretreat the biomass slurry by breaking it up or drying it. However, it is also possible to employ pretreated cell material in the process according to the invention. Surprisingly, as discussed below, it was discovered that when a heated biomass is suspended in a solution of an oxidizing agent such as 5-50% hydrogen peroxide having a final concentration of 0.01 - 30.0 percent volume/volume the cell wall of PHA-producing microorganisms having an overall PHA concentration of greater than 30% can be selectively disrupted with little damage to the PHA biopolymer. Applicant theorizes, without wishing to be bound thereto, that as the PHA concentration increases within a PHA-producing microorganisms the cell wall becomes both strained and more vulnerable to lysis. In addition, it is believed that the enzymes present in the biomass are deactivated as a result of the heating; consequently, when the oxidizing agent is added to the slurry, the native enzymes do not immediately decompose the oxidizing agent into harmless components. Many oxidizing agents when exposed to microorganisms will increase the permeability of the cell wall by disrupting sulphydryl (-SH) and sulphur (-S) bonds creating "holes" in the cell wall that typically do not result in lysis. However, when PHA-producing cells are engorged with PHA, and the oxidizing agent is not readily decomposed, the cells are lysed according to the present invention. The inventors theorize that the cell wall becomes strained as a result of the PHA content and as "holes" appear, as a result of the oxidizing agent, the cell wall begins to fail and spill out the contents of the cell. The discovery by the present inventors that the PHA-producing cells could be oxidized by the oxidizing agents disclosed herein at the concentrations used was surprising, as it is a finding quite contradictory to the well known principle that cell walls are not vulnerable to lysis when exposed to the oxidizing agents disclosed herein at the concentrations used. Thus, the method of this invention displays a number of notable advantages in comparison to the prior art. The PHA-producing cells are able to be disrupted while preserving the maximum original characteristics of the PHA biopolymer located inside the cell.

The solids content of the biomass slurry, which preferably contains water, is then adjusted to approximately, and preferably, 10-30 g/L, 30-50 g/L, 50-70 g/L, 70-200 g/L, or 200-500 g/L total solids and the cells are treated to inactivate metabolic and enzymatic activity to prevent degradation of both (i) the PHA biopolymer and (ii) the oxidizing agent. The preferred method to inactivate metabolic and enzymatic activity of the cells is to hold the biomass slurry at a temperature in the range of 50-80°C, 80-95°C, 95-99°C, or overlapping ranges therein, including for various time durations, including 1-5, 10, 30, 60, 120, 240, 480, and 960 minutes, including overlapping durations therein. This temperature assures that the cells are metabolically and enzymatically inactive without lysing the cells. Other methods known to selectively kill cells without lysis are described in Lawlis, Jr., *et. al.,* (U.S. Patent No. 5,378,621) describing the use adding about 1 to 2% by weight of acetic acid. Other methods may include the addition of detergents or caustic chemicals as is well known in the art.

The inactivated cells are then selectively lysed by mixing in an oxidizing agent, and water which is stirred at temperatures greater than 40°C, 50°C, 60°C, 70°C, 72°C, 74°C, 77°C, 83°C, 85°C, 89°C, 92°C, 95°C, or 99°C and preferably great than 60°C, 73°C, 87°, or 94°C for a period of time sufficient to lyse the inactive cells which is typically 0.5 - 24 hours. The mixture is preferably stirred with the aid of mixers, for example with the aid of static mixers. The oxidizing agent is added to achieve a final concentration of 0.5 - 30.0% volume/volume. The oxidizing agents useful in the present invention are preferably, non-gaseous at room temperature, have an oxidation-reduction potential (EO) of more than 1.4 volts, a pH between 3 and 11, and a molar mass of less than 250g/mol. The oxidizing agent may be for example one or more types each selected from organic and inorganic peroxides, such as but not limited to sodium peroxide (Na₂O₂), hydrogen peroxide (H₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate. It is preferred, however, to use hydrogen peroxide either as such or as a compound which produces hydrogen peroxide *in situ* or acts as an equivalent thereof, suitably a percarboxylic acid, for example peracetic acid, a perborate or a percarbonate. It is preferred however to introduce a 25-45 percent, and more preferably 30-40 percent and ideally 30-35 percent hydrogen peroxide to achieve a final concentration of 0.5 (0.5 to 3) percent volume/volume (0.1N H₂O₂).

It may be desirable to introduce the oxidizing agent continuously or intermittently during the process rather than introducing the whole amount at the beginning thereby minimizing the losses of oxidizing agent due to thermal decomposition.

After the cell walls have been adequately disrupted the suspension mat undergoe a solid/liquid separation to obtain solids comprising exposed PHA bioploymer *(e.g.,* as granules), proteins, peptides, amino acids and other cell residues (referred to herein as "NPCM") and an aqueous phase. While it is preferred to separate out the aqueous/solid phases by filtration or gravity separation, other separation processes which can be employed here are decanting and centrifugation of the NPCM containing the PHA biopolymer from the aqueous phase. The recovery and purification of the PHA from the NPCM can then be performed by a variety of different extraction methodologies, whether a solvent or aqueous extraction methodology is used one skilled in the art will appreciate that the initial step of lysing the cells is so gentle that PHA degradation is avoided, thus resulting in product having average molecular weight of PHAs typically range from about 10kDa - 3,000kDa, and more preferably in the range of 200kDa - 1,000kDa and can account for up to approximately 90%, 95%, or 99% of the initial polymer available within the starting biomass. Purity of PHA from the oxidized lipid degradation products can be increased further through volatilization, as well as aqueous or solvent washing steps.

It should be noted that the process for extracting PHA biopolymer may comprise different steps, and also as having varied designs, which are not explicitly mentioned. Examples of such are one or more separation steps, concentration, stirring, controlling temperature and/or controlling pH, etc. Moreover, the design of the equipment used may vary, and the present invention, as according to the claims, should be seen as embodying different forms of equipment.

The present invention will become more clear from consideration of the following examples which are set forth to further illustrate the principles of the invention and are not intended, in any way, to be limitative thereof.

### EXAMPLES

### EXAMPLE 1

A culture of *Cupriavidus, Methylocystis, Methylsinus, Methylococcus, Halomonas, Zobellella, Pseudomonas, Bacillus,* and/or *Chromobacterium strains* was grown in batch culture in an aqueous medium on a carbon source comprising methane to give a culture containing 25-200 g/l of cells containing 30-90% of a 3-polyhydroxybutyrate.

These PHA containing cells were then heated to 60°C - 95°C and a hydrogen peroxide solution was added to a final concentration of 0.5-15% v/v. The mixture is then stirred for an additional 1-24 hours with maintenance of the same temperature. At the end of this time the solution undergoes a solid/liquid extraction and the PHA biopolymers solids were recovered by filtration, washed and dried.

Analysis of the polymer product for impurities indicated 0.5%. The polymer product was thus considered to be 99.5% pure poly-3-hydroxybutyrate.

### EXAMPLE 2

A culture of *Cupriavidus, Methylocystis, Methylsinus, Methylococcus, Halomonas, Zobellella, Pseudomonas, Bacillus,* and/or *Chromobacterium strains* was grown in batch culture in an aqueous medium on a carbon source comprising methane to give a culture containing 25-200 g/l of cells containing 30-90% of a 3-polyhydroxybutyrate.

These PHA containing cells were then heated 60°C - 95°C. A peracetic acid solution is added to a final pH of 3. The mixture is then stirred for an additional 1-24 hours with maintenance of the same temperature. At the end of this time the solution undergoes a solid/liquid extraction and the PHA biopolymers solids were recovered by gravity separation, washed and dried.

Having disclosed several embodiments, it will be recognized by those of skill in the art that various modifications, alternative constructions, and equivalents may be used without departing from the spirit of the disclosed embodiments. Additionally, a number of well-known processes and elements have not been described in order to avoid unnecessarily obscuring the present invention. Accordingly, the above description should not be taken as limiting the scope of the invention.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included.

As used herein and in the appended claims, the singular forms "a", "an", and "the" include plural referents unless the context clearly dictates otherwise. Thus, for example, reference to "a process" includes a plurality of such processes and reference to "the dielectric material" includes reference to one or more dielectric materials and equivalents thereof known to those skilled in the art, and so forth.

Also, the words "comprise," "comprising," "include," "including," and "includes" when used in this specification and in the following claims are intended to specify the presence of stated features, integers, components, or steps, but they do not preclude the presence or addition of one or more other features, integers, components, steps, acts, or groups.
The invention contains the following embodiments:
1. A method for decreasing the production of foam when adding an oxidizing agent to a cellular biomass, comprising:
   heating the cellular biomass to a temperature sufficient to deactivate any enzymes present in the cellular biomass that adversely interact with said oxidizing agent; and
   adding said oxidizing agent to said heated cellular biomass forming a heated suspension to release the cellular content of said cellular biomass.
2. The method of embodiment 1, wherein the cellular biomass is heated to a temperature in the range of 80°C - 130°C.
3. The method of embodiment 1, wherein said oxidizing agent comprises a non-gaseous compound at room temperature having an oxidation-reduction potential (E_{O}) of more than 1.4 volts, a pH of more than 3 and less than 11, and a molar mass of less than 250g/mol.
4. The method of embodiment 3, wherein said oxidizing agent comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.
5. The method of embodiment 1, wherein said heated suspension of cellular biomass in contact with said oxidizing agent contains a final concentration of 0.1 % - 10 % of said oxidizing agent.
6. The method of embodiment 1, wherein said cellular biomass comprises polyhydroxyalkanoate (PHA) containing microorganisms.
7. The method of embodiment 6, wherein said PHA-containing microorganisms contain greater than 25% PHA by dry cell weight.
8. The method of embodiment 6, further comprising the step of separating and purifying said PHA from the cellular biomass and said cellular content.
9. The method of embodiment 8, wherein said PHA has a molecular weight of about 100kDa - 3,000kDa and a purity greater than 95.5%.
10. A method for providing a source of polyhydroxyalkanoate (PHA) for further purification, comprising:
   heating a suspension of microorganisms containing PHA;
   exposing said heated suspension of PHA-containing microorganisms to an oxidizing agent; and
   maintaining said heated suspension of PHA-containing microorganisms in contact with said oxidizing agent for a period of time and at a temperature sufficient to condition and lyse said microorganisms thereby releasing PHA and non-polymer cell mass (NPCM).
11. The process of embodiment 10, wherein said heated suspension of PHA-containing microorganisms in contact with said oxidizing agent contains a final concentration of 0.1 % - 10 % of said oxidizing agent.
12. The process of embodiment 11, wherein said heated suspension of PHA-containing microorganisms in contact with said oxidizing agent contains a final concentration of 0.5% - 6.0% of said oxidizing agent.
13. The process of embodiment 11, wherein said oxidizing agent comprises a non-gaseous compound at room temperature having an oxidation-reduction potential (E_{O}) of more than 1.4 volts, a pH of more than 3 and less than 11, and a molar mass of less than 250g/mol.
14. The process of embodiment 11, wherein said oxidizing agent comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.
15. The process of embodiment 12, wherein said oxidizing agent comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.
16. The process of embodiment 13, wherein said oxidizing agent comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.
17. The process of embodiment 10, wherein said temperature and time sufficient to condition and lyse said microorganisms and dissolve said NPCM is in the range of 80°C - 130°C for greater than 1 hour.
18. The process of embodiment 17, wherein said temperature and time sufficient to condition and lyse said microorganisms and dissolve said NPCM is in the range of 80°C - 90°C for 2 - 3 hours.
19. The process of embodiment 10, wherein said temperature and time sufficient to condition and lyse said microorganisms and dissolve said non-polymer cell mass is greater than 87°C for approximately 2.5 hours.
20. The process of embodiment 10, wherein no organic solvent is used in the extraction process.
21. The process of embodiment 10, wherein said polyhydroxyalkanoates (PHAs) is selected from the group consisting of poly(3-hydroxypropionate) (PHP or P3HP), poly(3-hydroxybutyrate) (PHB or P3HB), poly(4-hydroxybutyrate) (P4HB), poly(3-hydroxyvalerate) (PHV or P3HV), poly(4-hydroxyvalerate) (P4HV), poly(5-hydroxyvalerate) (P5HV), poly(3-hydroxyhexanoate) (PHHx or P3HHx), poly(3-hydroxyoctanoate) (PHO, or P3HO), poly(3-hydroxydecanoate) (PHD or P3HD), poly(3-hydroxyundecanoate) (PHU, P3HU), or other short- or medium-chain length, saturated or unsaturated PHAs; or polylactic acid (PLA); or their copolymers or any combinations thereof.
22. The process of embodiment 10, wherein suspension of microorganisms containing greater than 25 % PHA by dry cell weight.
23. An aqueous extraction process of polymers from a culture of microorganisms, comprising:
   contacting a suspension of microorganisms with an oxidizing agent wherein said oxidizing agent has a final concentration of approximately 0.5% - 6.0%;
   maintaining said suspension of microorganisms in contact with said oxidizing agent at a temperature greater than about 87°C for approximately 2.5 hours to lyse open said microorganisms freeing said polymer and dissolve said non-polymer cell mass.
24. The process of embodiment 23, wherein said freed polymer has a molecular weight of about 100kDa - 3,000kDa.
25. The process of embodiment 23, wherein said oxidizing agent comprises a non-gaseous compound at room temperature having an oxidation-reduction potential (E_{O}) of more than than 1.4 volts, a pH ofmore than 3 and less than 11, and a molar mass of less than 250g/mol.
26. The process of embodiment 25, wherein said oxidizing agent comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.
27. An aqueous extraction process of polymers from a culture of microorganisms, comprising:
   contacting a suspension of microorganisms with a non-gaseous compound at room temperature having an oxidation-reduction potential (E_{O}) of more than than 1.4 volts, a pH of more than 3 and less than 11, and a molar mass of less than 250g/mol wherein said non-gaseous compound has a final concentration of approximately 0.5% - 6.0%;
   maintaining said suspension of microorganisms in contact with said non-gaseous compound at a temperature greater than about 87°C for approximately 2.5 hours to lyse open said microorganisms freeing said polymer and dissolve said non-polymer cell mass.
28. The process of embodiment 27, wherein said non-gaseous compound comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.
29. The lysis solution of embodiment 27, wherein said said non-gaseous compound comprises a 30 - 35 % hydrogen peroxide solution having a final concentration of 0.4 - 4% volume/volume.

## Claims

1. An aqueous extraction process of polymers from a culture of microorganisms, comprising:
contacting a suspension of microorganisms with an oxidizing agent wherein said oxidizing agent has a final concentration of approximately 0.5% - 6.0%;
maintaining said suspension of microorganisms in contact with said oxidizing agent at a temperature greater than about 87°C for approximately 2.5 hours to lyse open said microorganisms freeing said polymer and dissolve said non-polymer cell mass.

2. The process of claim 1, wherein said freed polymer has a molecular weight of about 100kDa - 3,000kDa.

3. The process of claim 1, wherein said oxidizing agent comprises a non-gaseous compound at room temperature having an oxidation-reduction potential (E_{O}) of more than 1.4 volts, a pH of more than 3 and less than 11, and a molar mass of less than 250g/mol.

4. The process of claim 3, wherein said oxidizing agent comprises hydrogen peroxide (H₂O₂) and other inorganic peroxides, such as but not limited to sodium peroxide (Na₂O₂), sodium perborate (Na₂H₄B₂O₈), sodium percarbonate (Na₂H₃CO₆), and sodium persulfate (Na₂S₂O₈); chlorite, chlorate, metachloro perbenzoic acid (C₇H₅ClO₃) perchlorate, performic acid (CH₂O₃), peracetic acid (CH₃CO₃H), perchlorate (ClO₄), chlorine dioxide (ClO₂) and other analogous halogen compounds; permanganate compounds such as potassium permanganate; sodium perborate; potassium nitrate (KNO₃), sodium bismuthate; and cerium (IV) compounds such as ceric ammonium nitrate and ceric sulfate.

5. The process of any one of claims 1 to 4, wherein the oxidizing agent comprises a 30 - 35 % hydrogen peroxide solution having a final concentration of 0.4 - 4% volume/volume.
